# EUROPEAN PATENT APPLICATION

(11) **EP 4 567 103 A1**
(43) Date of publication of application: **11.06.2025**
(21) Application number: 23307131.5
(22) Date of filing: 04.12.2023
(51) Int. Cl.: C12N 5/0783, A61K 39/00

(54) **USE OF PYRUVATE FOR POTENTIATING NK CELLS**

(71) Applicant: LVMH Recherche, 45800 Saint Jean de Braye (FR); Institut National de la Santé et de la Recherche Médicale, 75013 Paris (FR); Centre National de la Recherche Scientifique, 75016 Paris (FR); Université Claude Bernard Lyon 1, 69100 Villeurbanne (FR); Ecole Normale Supérieure de Lyon, 69007 Lyon (FR)
(72) Inventor: Nizard, Carine, 45800 Saint Jean de Braye (FR); Kern, Nicolas, 45800 Saint Jean de Braye (FR); Bulteau, Anne-Laure, 45800 Saint Jean de Braye (FR); Marçais, Antoine, 69009 Lyon (FR); Walzer, Thierry, 69480 Pommiers (FR)
(74) Representative: Bandpay & Greuter

(57) **Abstract**

The present invention relates to the use of pyruvate for potentiating natural killer (NK) cells in vitro. The present invention also relates to a method for treating in vitro NK cells, comprising the steps of providing NK cells from a subject; incubating the NK cells in the presence of pyruvate at a concentration of from 0.35 to 10 mM, thereby potentiating the NK cells; and collecting the potentiated NK cells.

## Description

### Technical field

The present invention relates to the use of pyruvate for potentiating NK cells, to a method of treating NK cells *in vitro* with pyruvate, and a pharmaceutical composition comprising NK cells potentiated by pyruvate.

### Technical background

Natural killer (NK) cells are innate large granular lymphocytes that display immediate effector functions upon recognition of transformed or infected cells, accounting for 5% to 20% of the total number of peripheral blood lymphocytes. The effector functions include, for example, cytokine secretion to modulate the activities of other immune cells, and degranulation toward target cells. Although these functions rely on high energetic expenditure, the involved environmental nutrients and how they sustain these functions are unknown.

There is a rapidly expanding area of research that holds great potential for improving NK cell-based immunotherapies (also known as cell therapy).

For example, document CN104152413A discloses a culture system for efficient amplification of NK cells, comprising a RPMI 1640 medium, sodium bicarbonate, human serum albumin, transferrin, linoleic acid, oleic acid, palmitic acid, sodium pyruvate, human recombinant insulin, non-essential amino acids, 2-mercaptoethanol and interleukin-2.

Document WO2023075346A1 discloses a method for enhancing the anticancer function of NK cells through the regulation of an antioxidant mechanism, comprising removing the *keap1* gene or culturing the NK cells in a medium containing an antioxidant material.

However, in order to use NK cells in a cell therapy, it is necessary to find an effective way to enhance NK cells' activities.

Thus, there exists a continuous need for an effective method which makes it possible to potentiate (i.e., improve or increase the effector functions of) NK cells.

### Summary of the invention

It is a first object of the invention to provide the use of pyruvate for potentiating natural killer (NK) cells in vitro.

In some embodiments, the NK cells are potentiated by incubation in the presence of pyruvate at a concentration of from 0.35 to 10 mM, preferably 1 to 10 mM, more preferably from 1 to 5 mM.

In some embodiments, the NK cells are derived from a subject, preferably from peripheral blood mononuclear cells of the subject.

It is a second object of the invention to provide a method for treating in vitro NK cells, comprising the steps of providing NK cells from a subject; incubating the NK cells in the presence of pyruvate at a concentration of from 0.35 to 10 mM, thereby potentiating the NK cells; and collecting the potentiated NK cells.

In some embodiments of the method, incubating the NK cells in the presence of pyruvate is carried out for a period of from about 2 to 48 hours.

In some embodiments, the method further comprises incubating the NK cells in the presence of activating cytokines, the cytokines preferably comprising IL-12, IL-15, IL-18 and/or functional fragments thereof, preferably for a period of from about 2 to 48 hours, preferably from about 16 to 18 hours.

In some embodiments of the method, the step of providing NK cells from a subject comprises isolating the NK cells from peripheral blood mononuclear cells of the subject.

In some embodiments of the method, the subject suffers from a disease selected from cancer, an autoimmune disorder, an immune system disorder, and an infectious disease such as bacterial disease and viral disease.

In some embodiments of the method, the method further comprises a step of expressing a chimeric antigen receptor on the surface of the NK cells prior to, at the same time as, or after the step of incubation.

It is a third object of the invention to provide a pharmaceutical composition comprising NK cells obtained by the above method and a pharmaceutically acceptable carrier.

In some embodiments of the pharmaceutical composition, the NK cells have an intracellular ATP concentration of 1 to 10 fmol/cell.

In some embodiments of the pharmaceutical composition, the NK cells express cytokines, preferably IFN-γ and MIP1-β, and/or surface cytotoxic markers, preferably CD107a, more preferably at least 3% of the NK cells expressing the IFN-γ, and/or at least 40% of the NK cells expressing MIP1-β, and/or at least 5% of the NK cells expressing CD107a.

It is a fourth object of the invention to provide a pharmaceutical composition comprising NK cells obtained by the above method and a pharmaceutically acceptable carrier, for use in the treatment of cancer, an autoimmune disorder, an immune system disorder, and an infectious disease such as bacterial disease and viral disease.

In some embodiments of the pharmaceutical composition for use, the NK cells have an intracellular ATP concentration of 1 to 10 fmol/cell or higher.

In some embodiments of the pharmaceutical composition for use, the pharmaceutical composition is intended for administration to the subject, preferably via intravenous infusion, and wherein preferably the NK cells are derived from the same subject.

The present invention enables to address the need mentioned above. In particular, the invention makes it possible to effectively potentiate NK cells.

This is achieved by the use of pyruvate for potentiating NK cells *in vitro.*

The present inventors have surprisingly discovered that supra-physiological pyruvate concentrations can enhance NK cell effector functions in a dose-dependent manner.

Thus, the use of extracellular pyruvate can potentiate the effector functions of the NK cells, resulting in, for example, increased cytokine production and increased cytotoxicity.

### Brief description of the drawings

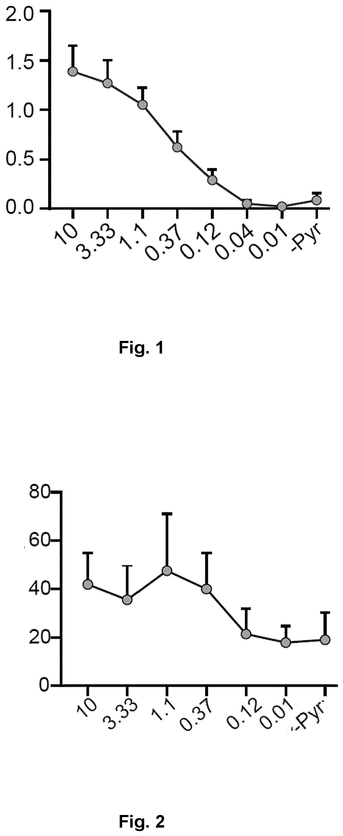

**Figure 1** shows the variation of ATP concentration in NK cells incubated in the presence of varying concentrations of pyruvate. The intracellular ATP concentration (arbitrary unit) can be read on y-axis, and the pyruvate concentration (mM) can be read on x-axis. "-Pry" indicates the absence of pyruvate.
**Figure 2** shows the effect of varying concentrations of pyruvate on the cytotoxicity of NK cells. The K562 lysis rate (%) can be read on y-axis, and the pyruvate concentration (mM) can be read on x-axis. "-Pry" indicates the absence of pyruvate.

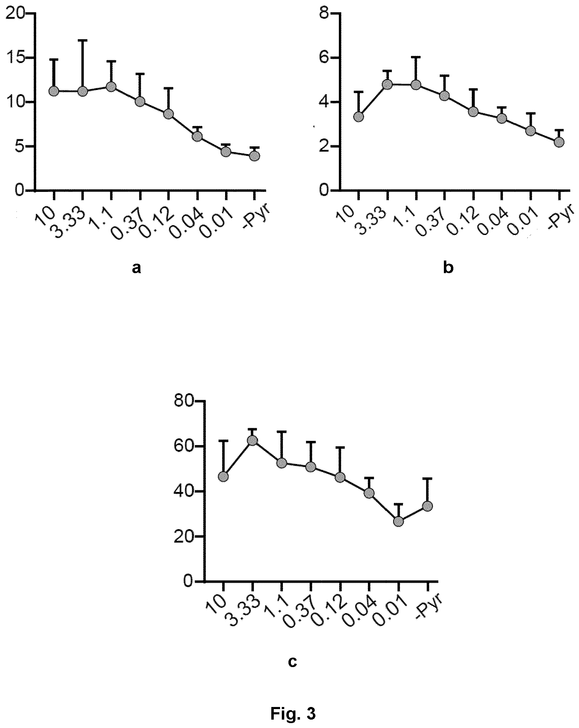

**Figure 3** shows the effect of varying concentrations of pyruvate on the expression of markers of NK cells stimulated with a target cell line (K562). The percentage of NK cells expressing the marker can be read on y-axis (CD107a⁺ NK cells (%) in **Figure 3a**, IFN-γ⁺ NK cells (%) in **Figure 3b**, MIP1-β⁺ NK cells (%) in **Figure 3c**). The pyruvate concentration (mM) can be read on x-axis. "-Pry" indicates the absence of pyruvate.

### Detailed description

The invention will now be described in more detail without limitation in the following description.

The term "stimulate (or stimulation of) NK cells" as used herein means activate NK cells.

The term "activate (or activation of) NK cells" as used herein means that, upon activation, the NK cells display their effector functions (such as cytokine secretion and degranulation), which can be characterized by the expression of several markers such as CD107a, IFN-γ and/or MIP1-β.

The term "potentiate (or potentiation of) NK cells" as used herein means improving or increasing the magnitude of the effector functions of NK cells (i.e., activated NK cells), which can be characterized by an increased expression of the above markers after stimulation, for example, with a third party tumor cell line, relative to non-potentiated NK cells after stimulation, for example, with a third party tumor cell line.

The term "*in vitro*" as used herein refers to a process performed or taking place in an artificial environment (e.g., in a test tube, reaction vessel, cell culture) rather than within a multi-cellular organism.

The term "pyruvate" as used herein refers to a compound having a structure CH₃-C(O)-COOH and salts thereof, and is an intermediate in several metabolic pathways throughout a cell. Pyruvate includes pyruvic acid and the anionic form provided together with a counterion, such as sodium, potassium, calcium, preferably sodium.

The term "incubate (or incubation)" as used herein means keeping the cells in conditions suitable for continued development and/or growth. The term "culture" may be used interchangeably.

The term "derived from" as used herein means that an element (e.g., NK cells) originates from the specified source, in other words, has been obtained (directly or indirectly) from the specified source.

The term "physiological concentration (of pyruvate)" as used herein refers to the concentration (of pyruvate) under normal conditions found in a subject. The physiological concentration of pyruvate present in blood is relatively low (40 to 150 µM). The term "supra-physiological concentration (of pyruvate)" refers to a concentration (of pyruvate) above the physiological concentration, for example from 0.35 to 10 mM.

The term "extracellular pyruvate" as used herein means pyruvate which is present outside the plasma membrane of a cell (e.g., NK cell), or exogenous pyruvate. The term "exogenous pyruvate" as used herein, refers to pyruvate produced outside of the cell (e.g., NK cell). The terms "extracellular pyruvate" and "exogenous pyruvate" may be used herein interchangeably.

The term "cytokines" as used herein refers to proteins involved in cell communication and signaling, such as interleukins and chemokines. The term "activating cytokines" refer to such cytokines that can activate NK cells.

The term "functional fragments (of interleukins)" as used herein refers to truncated polypeptides (and variants thereof) as compared to the naturally occurring interleukins, the truncated polypeptides being capable of possessing (at least to some extent) the same activity as the naturally occurring interleukins (e.g., activation of NK cells).

The term "chimeric antigen receptor (CAR)" as used herein refers to a protein that specifically recognizes a target antigen (e.g., a target antigen on a cancer cell) via its antigen recognition domain derived from a monoclonal antibody. Upon binding to a target antigen, a CAR can activate immune cells to attack and destroy cells carrying that antigen.

The term "pharmaceutically acceptable" as used herein can describe substances or components that do not cause unacceptable losses of pharmacological activity or unacceptable adverse side effects.

### Use of pyruvate for potentiating NK cells

The present invention provides the use of pyruvate for potentiating natural killer (NK) cells *in vitro.*

The NK cells may be potentiated by incubation with the pyruvate at a concentration of from 0.35 to 10 mM. The pyruvate concentration may be for example from 0.35 to 0.5 mM, 0.5 to 1 mM, 1 to 3 mM, from 3 to 5 mM, from 5 to 7 mM, from 7 to 9 mM, and from 9 to 10 mM. The pyruvate concentration may be preferably 1 to 10 mM, and more preferably 1 to 5 mM.

The above range is higher than the physiological concentration of pyruvate present in blood (i.e., 40 to 150 µM) and thus may be considered as supra-physiological concentration.

It is to be noted that conventional culture media supplemented with 10% Fetal bovine serum (FBS, also known as fetal calf serum (FCS)) may typically contain 10 µM pyruvate in total.

The conditions for the incubation of NK cells in the presence of pyruvate may be appropriately selected, for example, by following a conventional NK cell culture procedure.

In some embodiments, the incubation of the NK cells may be carried out for a period of from about 2 to 48 hours. For example, the incubation of the NK cells may be carried out for a period of from about 2 to 5 hours, from about 5 to 10 hours, from about 10 to 20 hours, from about 20 to 30 hours, from about 30 to 40 hours, or from about 40 to 48 hours.

In some embodiments, the incubation of the NK cells may be carried out at approximately 37°C.

In some embodiments, the incubation of the NK cells may be carried out in 1 to 10% CO₂, preferably 3 to 7% CO₂. For example, the incubation of the NK cells may be carried out in 3% CO₂, 4% CO₂, 5% CO₂, 6% CO₂, or 7% CO₂. By "X%CO₂" is meant that the percentage of CO₂ relative to the total volume of gases in the atmosphere (for example, the atmosphere inside an incubator) is X% by volume.

In some embodiments, the initial incubation concentration of NK cells may be from 1 × 10⁴ to 1 × 10⁶ cells/mL.

The concentration of NK cells may be measured by a conventional cell counting technique, such as hemocytometry, flow cytometry, or counting by image analysis.

The medium for incubation of the NK cells may be any commercially available medium appropriate for the cultivation and maintenance of NK cells, such as RPMI-1640 medium, or Iscove's Modified Dulbecco's Medium (IMDM). For example, the RPMI-1640 medium comprises amino acids (glycine, L-arginine, L-asparagine, L-aspartic acid, L-cystine 2HCl, L-glutamic acid, L-glutamine, L-histidine, L-hydroxyproline, L-isoleucine, L-leucine, L-lysine hydrochloride, L-methionine, L-phenylalanine, L-proline, L-serine, L-threonine, L-tryptophan, L-tyrosine disodium salt dihydrate, L-valine); vitamins (biotin, choline chloride, D-calcium pantothenate, folic acid, niacinamide, para-aminobenzoic acid, pyridoxine hydrochloride, riboflavin, thiamine hydrochloride, vitamin B12, i-inositol); inorganic salts (calcium nitrate (Ca(NO₃)₂ 4H₂O), magnesium sulfate (MgSO₄) (anhyd.), potassium chloride (KCl), sodium bicarbonate (NaHCO₃), sodium chloride (NaCl), sodium phosphate dibasic (Na₂HPO₄) anhydrous); D-glucose (dextrose); glutathione (reduced); phenol red.

The incubation of the NK cells may be carried out in an incubator.

The NK cells may be derived from a subject.

The subject may be an animal subject, including a mammal, such as horses, cows, dogs, cats, sheep, pigs, mice, rats, monkeys, hamsters, guinea pigs, and humans. Preferably, the subject is a human subject.

The NK cells may be derived from peripheral blood or umbilical cord blood, preferably peripheral blood of the subject.

More preferably, NK cells may be derived from peripheral blood mononuclear cells (PBMCs). By "PBMC" is meant any peripheral blood cell having a round nucleus, including lymphocytes (T cells, B cells, NK cells) and monocytes.

The use of pyruvate of the invention may be used in applications such as research studies to understand immune responses or in immune cell therapy for treating certain medical conditions.

### Method for treating in vitro NK cells

The present invention also provides a method for treating *in vitro* natural killer (NK) cells.

The method comprises the steps of:
- providing NK cells from a subject;
- incubating the NK cells in the presence of pyruvate at a concentration of from 0.35 to 10 mM, thereby potentiating the NK cells; and
- collecting the potentiated NK cells.

The subject may be as described above in relation to the use of pyruvate for potentiating NK cells.

In some embodiments, the subject may suffer from a disease selected from cancer, an autoimmune disorder, an immune system disorder, and an infectious disease such as bacterial disease and viral disease.

Examples of the cancer include hematological cancer or a cancer with a solid tumor such as leukemia; advanced non-small cell lung cancer; breast cancer; colon carcinoma; gastric carcinoma; Hodgkin's disease; lymphoma tumors; glioma; lung cancer; melanoma; melanoma; metastatic breast carcinoma; metastatic melanoma; metastatic renal cell carcinoma; multiple myeloma; neuroblastoma; non-Hodgkin's lymphoma; osteosarcoma; renal cell carcinoma; soft-tissue sarcoma; renal cell carcinoma; or ovarian cancer.

The examples of leukemia may include acute myeloid (or myelogenous) leukemia (AML); chronic myeloid (or myelogenous) leukemia (CML); acute lymphocytic (or lymphoblastic) leukemia (ALL); chronic lymphocytic leukemia (CLL); hairy cell; T-cell prolymphocytic; or juvenile myelomonocytic leukemia.

Additional examples of cancers can be Adrenocortical Carcinoma; AIDS-Related Cancers; Kaposi Sarcoma (Soft Tissue Sarcoma); AIDS-Related Lymphoma (Lymphoma); Primary Central Nervous System (CNS) Lymphoma (Lymphoma); Anal Cancer; Appendix Cancer; Gastrointestinal Carcinoid Tumors; Astrocytomas; Atypical Teratoid/Rhabdoid Tumor, Childhood, Central Nervous System (Brain Cancer); Basal Cell Carcinoma of the Skin; Bile Duct Cancer; Bladder Cancer; Bone Cancer (including Ewing Sarcoma and Osteosarcoma and Malignant Fibrous Histiocytoma); Brain Tumors; Breast Cancer; Bronchial Tumors; Burkitt Lymphoma; Carcinoid Tumor (Gastrointestinal); Childhood Carcinoid Tumors; Cardiac (Heart) Tumors; Central Nervous System cancer; Atypical Teratoid/Rhabdoid Tumor, Childhood (Brain Cancer); Embryonal Tumors, Childhood (Brain Cancer); Germ Cell Tumor, Childhood (Brain Cancer); Cervical Cancer; Cholangiocarcinoma; Bile Duct Cancer Chordoma; Chronic Myeloproliferative Neoplasms; Colorectal Cancer; Craniopharyngioma (Brain Cancer); Cutaneous T-Cell; Ductal Carcinoma In Situ (DCIS); Embryonal Tumors, Central Nervous System, Childhood (Brain Cancer); Endometrial Cancer (Uterine Cancer); Ependymoma, Childhood (Brain Cancer); Esophageal Cancer; Esthesioneuroblastoma; Ewing Sarcoma (Bone Cancer); Extracranial Germ Cell Tumor; Extragonadal Germ Cell Tumor; Eye Cancer; Intraocular Melanoma; Retinoblastoma; Fallopian Tube Cancer; Fibrous Histiocytoma of Bone, Malignant, or Osteosarcoma; Gallbladder Cancer; Gastric (Stomach) Cancer; Gastrointestinal Carcinoid Tumor; Gastrointestinal Stromal Tumors (GIST) (Soft Tissue Sarcoma); Germ Cell Tumors; Central Nervous System Germ Cell Tumors (Brain Cancer); Childhood Extracranial Germ Cell Tumors; Extragonadal Germ Cell Tumors; Ovarian Germ Cell Tumors; Testicular Cancer; Gestational Trophoblastic Disease; Hairy Cell Leukemia; Head and Neck Cancer; Heart Tumors; Hepatocellular (Liver) Cancer; Histiocytosis, Langerhans Cell; Hodgkin Lymphoma; Hypopharyngeal Cancer; Islet Cell Tumors; Pancreatic Neuroendocrine Tumors; Kidney (Renal Cell) Cancer; Langerhans Cell Histiocytosis; Laryngeal Cancer; Leukemia; Lip and Oral Cavity Cancer; Liver Cancer; Lung Cancer (Non-Small Cell and Small Cell); Lymphoma; Male Breast Cancer; Malignant Fibrous Histiocytoma of Bone or Osteosarcoma; Melanoma; Melanoma, Intraocular (Eye); Merkel Cell Carcinoma (Skin Cancer); Mesothelioma, Malignant; Metastatic Cancer; Metastatic Squamous Neck Cancer with Occult Primary; Midline Tract Carcinoma Involving NUT Gene; Mouth Cancer; Multiple Endocrine Neoplasia Syndromes; Multiple Myeloma/Plasma Cell Neoplasms; Mycosis Fungoides (Lymphoma); Myelodysplastic Syndromes, Myelodysplastic/Myeloproliferative Neoplasms; Myeloproliferative Neoplasms; Nasal Cavity and Paranasal Sinus Cancer; Nasopharyngeal Cancer; Neuroblastoma; Non-Hodgkin Lymphoma; Non-Small Cell Lung Cancer; Oral Cancer, Lip or Oral Cavity Cancer; Oropharyngeal Cancer; Ovarian Cancer Pancreatic Cancer; Pancreatic Neuroendocrine Tumors (Islet Cell Tumors); Papillomatosis; Paraganglioma; Paranasal Sinus and Nasal Cavity Cancer; Parathyroid Cancer; Penile Cancer; Pharyngeal Cancer; Pheochromocytoma; Pituitary Tumor; Plasma Cell Neoplasm/Multiple Myeloma; Pleuropulmonary Blastoma; Pregnancy and Breast Cancer; Primary Peritoneal Cancer; Prostate Cancer; Rectal Cancer; Recurrent Cancer Renal Cell (Kidney) Cancer; Retinoblastoma; Rhabdomyosarcoma, Childhood (Soft Tissue Sarcoma); Salivary Gland Cancer; Sarcoma; Childhood Rhabdomyosarcoma (Soft Tissue Sarcoma); Childhood Vascular Tumors (Soft Tissue Sarcoma); Ewing Sarcoma (Bone Cancer); Uterine Sarcoma; Sèzary Syndrome (Lymphoma); Skin Cancer; Small Intestine Cancer; Squamous Cell Carcinoma of the Skin; Stomach (Gastric) Cancer; T-Cell Lymphoma, Cutaneous; Lymphoma; Mycosis Fungoides and Sèzary Syndrome; Testicular Cancer; Throat Cancer; Nasopharyngeal Cancer; Oropharyngeal Cancer; Hypopharyngeal Cancer; Thymoma and Thymic Carcinoma; Thyroid Cancer; Thyroid Tumors; Transitional Cell Cancer of the Renal Pelvis and Ureter (Kidney (Renal Cell) Cancer); ; Urethral Cancer; Uterine Cancer, Endometrial; Uterine Sarcoma; Vaginal Cancer; Vascular Tumors (Soft Tissue Sarcoma); Vulvar Cancer; or Wilms Tumor.

Examples of the autoimmune disorder and the immune system disorder include Achalasia; Addison's disease; Adult Still's disease; Agammaglobulinemia; Alopecia areata; Amyloidosis; Ankylosing spondylitis; Anti-GBM/Anti-TBM nephritis; Antiphospholipid syndrome; Autoimmune angioedema; Autoimmune dysautonomia; Autoimmune encephalomyelitis; Autoimmune hepatitis; Autoimmune inner ear disease (AIED); Autoimmune myocarditis; Autoimmune oophoritis; Autoimmune orchitis; Autoimmune pancreatitis; Autoimmune retinopathy; Autoimmune urticaria; Axonal & neuronal neuropathy (AMAN); Bald disease; Behcets disease; Benign mucosal pemphigoid; Bullous pemphigoid; Castleman disease (CD); Celiac disease; Chagas disease; Chronic inflammatory demyelinating polyneuropathy (CIDP); Chronic recurrent multifocal osteomyelitis (CRMO); Churg-Strauss Syndrome (CSS) or Eosinophilic Granulomatosis (EGPA); Cicatricial pemphigoid; Cogan's syndrome; Cold agglutinin disease; Congenital heart block; Coxsackie myocarditis; CREST syndrome; Crohn's disease; Dermatitis herpetiformis; Dermatomyositis; Devic's disease (neuromyelitis optica); Discoid lupus; Dressler's syndrome; Endometriosis; Eosinophilic esophagitis (EoE); Eosinophilic fasciitis; Erythema nodosum; Essential mixed cryoglobulinemia; Evans syndrome; Fibromyalgia; Fibrosing alveolitis; Giant cell arteritis (temporal arteritis); Giant cell myocarditis; Glomerulonephritis; Goodpasture's syndrome; Granulomatosis with Polyangiitis; Graves' disease; Guillain-Barre syndrome; Hashimoto's thyroiditis; Hemolytic anemia; Henoch-Schonlein purpura (HSP); Herpes gestationis or pemphigoid gestationis (PG); Hidradenitis Suppurativa (HS) (Acne Inversa); Hypogammalglobulinemia; IgA Nephropathy; IgG4-related sclerosing disease; Immune thrombocytopenic purpura (ITP); Inclusion body myositis (IBM); Interstitial cystitis (IC); Juvenile arthritis; Juvenile diabetes (Type 1 diabetes); Juvenile myositis (JM); Kawasaki disease; Lambert-Eaton syndrome; Leukocytoclastic vasculitis; Lichen planus; Lichen sclerosus; Ligneous conjunctivitis; Linear IgA disease (LAD); Lupus; Lyme disease chronic; Meniere's disease; Microscopic polyangiitis (MPA); Mixed connective tissue disease (MCTD); Mooren's ulcer; Mucha-Habermann disease; Multifocal Motor Neuropathy (MMN) or MMNCB; Multiple sclerosis; Myasthenia gravis; Myositis; Narcolepsy; Neonatal Lupus; Neuromyelitis optica; Neutropenia; Ocular cicatricial pemphigoid; Optic neuritis; Palindromic rheumatism (PR); PANDAS; Paraneoplastic cerebellar degeneration (PCD); Paroxysmal nocturnal hemoglobinuria (PNH); Parry Romberg syndrome; Pars planitis (peripheral uveitis); Parsonage-Turner syndrome; Pemphigus; Peripheral neuropathy; Perivenous encephalomyelitis; Pernicious anemia (PA); POEMS syndrome; Polyarteritis nodosa; Polyglandular syndromes type 1, 11, 111; Polymyalgia rheumatica; Polymyositis; Postmyocardial infarction syndrome; Postpericardiotomy syndrome; Primary biliary cirrhosis; Primary sclerosing cholangitis; Progesterone dermatitis; Psoriasis; Psoriatic arthritis; Pure red cell aplasia (PRCA); Pyoderma gangrenosum; Raynaud's phenomenon; Reactive Arthritis; Reflex sympathetic dystrophy; Relapsing polychondritis; Restless legs syndrome (RLS); Retroperitoneal fibrosis; Rheumatic fever; Rheumatoid arthritis; Sarcoidosis; Schmidt syndrome; Scleritis; Scleroderma; Sjögren's syndrome; Sperm & testicular autoimmunity; Stiff person syndrome (SPS); Subacute bacterial endocarditis (SBE); Susac's syndrome; Sympathetic ophthalmia (SO); Takayasu's arteritis; Temporal arteritis/Giant cell arteritis; Thrombocytopenic purpura (TTP); Tolosa-Hunt syndrome (THS); Transverse myelitis; Type 1 diabetes; Ulcerative colitis (UC); Undifferentiated connective tissue disease (UCTD); Uveitis; Vasculitis; Vitiligo; Vogt-Koyanagi-Harada Disease; or an autoinflammatory disease such as Familial Mediterranean Fever (FMF), neonatal Onset Multisystem Inflammatory Disease (NOMID), Tumor Necrosis Factor Receptor-Associated Periodic Syndrome (TRAPS), Deficiency of the Interleukin-1 Receptor Antagonist (DIRA), Behçet's Disease, or Chronic Atypical Neutrophilic Dermatosis with Lipodystrophy and Elevated Temperature (CANDLE).

The infectious disease may be any infectious disease that is caused by viral infections (e.g., cytomegalovirus, Epstein Barr virus, herpes simplex virus, human immunodeficiency virus), intracellular pathogens (e.g., Listeria monocytogenes ), bacterial infections, and fungal infections. Examples of the infectious disease may be Acute Flaccid Myelitis (AFM); Anaplasmosis; Anthrax; Babesiosis; Botulism; Brucellosis; Campylobacteriosis; Carbapenem-resistant Infection (CRE/CRPA); Chancroid; Chikungunya Virus Infection (Chikungunya); Chlamydia; Ciguatera (Harmful Algae Blooms (HABs)); Clostridium Difficile Infection; Clostridium Perfringens (Epsilon Toxin); Coccidioidomycosis fungal infection (Valley fever); coronavirus infections (including Severe Acute Respiratory Syndrome coronavirus 2 (SARS-CoV-2) infections and the corresponding disease known as coronavirus disease 2019 (COVID-19)); Creutzfeldt-Jacob Disease, transmissible spongiform encephalopathy (CJD); Cryptosporidiosis (Crypto); Cyclosporiasis; Dengue, 1,2,3,4 (Dengue Fever); Diphtheria; E. coli infection, Shiga toxin-producing (STEC); Eastern Equine Encephalitis (EEE); Ebola Hemorrhagic Fever (Ebola); Ehrlichiosis; Encephalitis, Arboviral or parainfectious; Enterovirus Infection, Non-Polio (Non-Polio Enterovirus); Enterovirus Infection, D68 (EV-D68); Giardiasis (Giardia); Glanders; Gonococcal Infection (Gonorrhea); Granuloma inguinale; Haemophilus Influenza disease, Type B (Hib or H-flu); Hantavirus Pulmonary Syndrome (HPS); Hemolytic Uremic Syndrome (HUS); Hepatitis A (Hep A); Hepatitis B (Hep B); Hepatitis C (Hep C); Hepatitis D (Hep D); Hepatitis E (Hep E); Herpes; Herpes Zoster, zoster VZV (Shingles); Histoplasmosis infection (Histoplasmosis); Human Immunodeficiency Virus/AIDS (HIV/AIDS); Human Papillomavirus (HPV); Influenza (Flu); Legionellosis (Legionnaires Disease); Leprosy (Hansens Disease); Leptospirosis; Listeriosis (Listeria ); Lyme Disease; Lymphogranuloma venereum infection (LGV); Malaria; Measles; Melioidosis; Meningitis, Viral (Meningitis, viral); Meningococcal Disease, Bacterial (Meningitis, bacterial); Middle East Respiratory Syndrome Coronavirus (MERS-CoV); Mumps; Norovirus; Paralytic Shellfish Poisoning (Paralytic Shellfish Poisoning, Ciguatera); Pediculosis (Lice, Head and Body Lice); Pelvic Inflammatory Disease (PID); Pertussis (Whooping Cough); Plague; Bubonic, Septicemic, Pneumonic (Plague); Pneumococcal Disease (Pneumonia); Poliomyelitis (Polio); Powassan; Psittacosis (Parrot Fever); Pthiriasis (Crabs; Pubic Lice Infestation); Pustular Rash diseases (Small pox, monkeypox, cowpox); Q-Fever; Rabies; Ricin Poisoning; Rickettsiosis (Rocky Mountain Spotted Fever); Rubella, Including congenital (German Measles); Salmonellosis gastroenteritis (Salmonella ); Scabies Infestation (Scabies); Scombroid; Septic Shock (Sepsis); Severe Acute Respiratory Syndrome (SARS); Shigellosis gastroenteritis (Shigella ); Smallpox; Staphyloccal Infection, Methicillin-resistant (MRSA); Staphylococcal Food Poisoning, Enterotoxin-B Poisoning (Staph Food Poisoning); Staphylococcal Infection, Vancomycin Intermediate (VISA); Staphylococcal Infection, Vancomycin Resistant (VRSA); Streptococcal Disease, Group A (invasive) (Strep A (invasive)); Streptococcal Disease, Group B (Strep-B); Streptococcal Toxic-Shock Syndrome, STSS, Toxic Shock (STSS, TSS); Syphilis, primary, secondary, early latent, late latent, congenital; Tetanus Infection, tetani (Lock Jaw); Trichomoniasis (Trichomonas infection); Trichonosis Infection (Trichinosis); Tuberculosis (TB); Tuberculosis (Latent) (LTBI); Tularemia (Rabbit fever); Typhoid Fever, Group D; Typhus; Vaginosis, bacterial (Yeast Infection); Vaping-Associated Lung Injury (e-Cigarette Associated Lung Injury); Varicella (Chickenpox); Vibrio cholerae (Cholera); Vibriosis (Vibrio ); Viral Hemorrhagic Fever (Ebola, Lassa, Marburg); West Nile Virus; Yellow Fever; Yersenia (Yersinia); or Zika Virus Infection (Zika).

In some embodiments, the step of providing NK cells from a subject may comprise isolating the NK cells from peripheral blood or umbilical cord blood, preferably peripheral blood of the subject.

The blood may be collected from the subject by venipuncture (i.e., injection of a needle to a peripheral vein or the like), or by apheresis (particularly cytapheresis, in which peripheral blood stem cells may collected, or leukapheresis, in which white blood cells may be collected).

The blood may be freshly collected blood, or may be blood that has been stored under appropriate conditions (at a temperature ranging from 4°C to 20°C).

In preferred embodiments, the NK cells may be provided from peripheral blood mononuclear cells (PBMCs) of the subject.

The isolation of PBMCs from the peripheral blood may be performed by a method well known in the art, using, for example density gradient centrifugation, flow cytometry or magnetic bead-based method.

The NK cells may be obtained from the PBMCs by a method well known in the art, for example using a commercially available NK cell isolation kit, or by flow cytometry or magnetic bead-based method.

The NK cells may be then rested under appropriate culture conditions, allowing them to recuperate and regain their full functionality after the isolation procedure.

During this resting phase, pyruvate may be added to the medium for the survival of the NK cells, for example at a concentration of from 0.01 to 5 mM, 0.1 to 5 mM, or 0.1 to 1 mM.

The NK cells may be rested for a period of from about 2 to 48 hours, for example overnight, at approximately 37°C.

The NK cells may be then activated, stored, or stored and then activated before the subsequent step of incubation, as explained below.

The NK cells may be stored before the subsequent step of incubation. For example, the NK cells may be stored in a standard laboratory refrigerator (at a temperature of from 2°C to 8°C) or in a laboratory freezer (at a temperature of from -20°C to -30°C) for a short-term storage, such as a few hours to overnight. Preferably, the NK cells may be stored in liquid nitrogen especially for a long-term storage (more than few weeks).

In the method of the invention, the NK cells are incubated in the presence of pyruvate at a concentration of from 0.35 to 10 mM, preferably 1 to 10 mM, more preferably from 1 to 5 mM.

The pyruvate concentration may be for example from 0.35 to 0.5 mM, from 0.5 to 1 mM, from 1 to 3 mM, from 3 to 5 mM, from 5 to 7 mM, from 7 to 9 mM, and from 9 to 10 mM. The pyruvate concentration may be preferably 1 to 10 mM, more preferably 1 to 5 mM.

The conditions and media for the incubation of NK cells in the presence of pyruvate may be as described above in relation to the use of pyruvate for potentiating NK cells.

Specifically, the incubation of the NK cells may be carried out for a period of from about 2 to 48 hours.

In some embodiments, the incubation of the NK cells may be carried out at approximately 37°C.

The initial incubation concentration of NK cells may be from 1 × 10⁴ to 1 × 10⁶ cells/mL.

The concentration of NK cells may be measured as described above.

The step of collecting the potentiated NK cells may comprise isolating and/or purifying the potentiated NK cells from the culture medium and other debris in the culture, using a conventional technique such as centrifugation, filtration, or magnetic bead isolation.

The collected NK cells may be subjected to quality control for the purity, viability and/or the capacity of effector functions (especially cytokine secretion), by way of, for example, microscopy, viability assay, flow cytometry, or fluorescence-activated cell sorting (FACS).

The collected NK cells may be then used for further applications. The applications include research studies to understand immune responses or in immune cell therapy for treating certain medical conditions.

The collected NK cells may be stored before such applications, as explained above.

The method may further comprise incubating the NK cells in the presence of activating cytokines and/or target cells, preferably activating cytokines.

The incubation of NK cells in the presence activating cytokines or target cells may be carried out for a period of from about 2 to 48 hours, preferably from about 16 to 18 hours. For example; the incubation of the NK cells in the presence activating cytokines or target cells may be carried out for a period of from about 2 to 5 hours, from about 5 to 10 hours, from about 10 to 20 hours, from about 20 to 30 hours, from about 30 to 40 hours, or from about 40 to 48 hours.

The activating cytokines may preferably comprise IL-12, IL-15, IL-18 and/or functional fragments thereof.

The activating cytokines may have a concentration that can trigger the activation of NK cells. The appropriate concentration may be selected depending on the cytokine to be used. For example the concentration may be from 5 to 100, preferably from 10 to 50 ng/mL for IL12; from 10 to 100, preferably from 50 to 80 ng/mL for IL-15; or from 10 to 100, preferably from 50 to 80 ng/mL for IL18.

In some embodiments, the activating cytokines may be fusion proteins comprising functional fragments of IL-12, IL-15, and/or IL-18.

The NK cells thus activated by activating cytokines may be cytokine-induced memory-like (CIML) NK cells, especially when the activating cytokines comprises a cytokine cocktail (the combination of IL-12, IL-15, and IL-18), their related family members, or functional fragments thereof.

It is known that CIML NK cells exhibit a memory-like phenotype, exhibiting enhanced response upon restimulation with the cytokines or triggering via activating receptors (e.g., target cells).

The NK cells may be incubated in the presence of both pyruvate and of the activating cytokines concurrently or separately, preferably concurrently. For example, when incubated concurrently, the NK cells may be incubated in the co-presence of pyruvate and activating cytokines; or the NK cells may first be incubated in the presence of pyruvate and then subsequently in the co-presence of pyruvate and activating cytokines; or the NK cells may first be incubated in the presence of activating cytokines and then subsequently in the co-presence of activating cytokines and pyruvate. Alternatively, when incubated separately, the NK cells may first be incubated in the presence of activating cytokines, the activating cytokines are removed, and then the NK cells may be incubated in the presence pyruvate; or the NK cells may first be incubated in the presence of pyruvate, the pyruvate is removed, and then the NK cells may be incubated in the presence activating cytokines.

The target cells may be any cells that are involved in a disease and can be targeted by NK cells, such as cancer cells (or cell line, such as leukemia cell line, for example K562 line), or infected cells.

The target cells may be co-cultured with NK cells in the presence of pyruvate.

The ratio of NK cells to the target cells may be, for example, from 1:1 to 30:1, preferably 5:1 to 20:1, and more preferably from 8:1 to 12:1.

The method may further comprise a step of expressing a chimeric antigen receptor (CAR) on the surface of the NK cells prior to, at the same time as, or after the step of incubation.

This step is especially advantageous when the collected NK cells are used for immune cell therapy. The antigen recognition by CAR-expressing NK cells is based on the binding of the antigen recognition domain of the CAR to target antigens, thus bypassing the need for complex immune recognition processes, such as MHC restriction, or co-receptor dependency. Thus, the expression of CARs on the surface of NK cells can further enhance the therapy efficacy.

The NK cells may be genetically engineered to express chimeric antigen receptors (CARs). Any suitable genetic engineering method may be used, for example, by way of transformation, transfection, transduction, or genome editing using a technology such as TALEN, CRISPR, or ZFN.

For example, NK cells may be incubated in the presence of both pyruvate and of activating cytokines, either concurrently or separately. Then, the CAR may be transduced via a viral vector (e.g., lentivirus) into the potentiated NK cells. Any commercially available transduction kit may be used. The transduction may be performed for a period of, for example, from 1 to 48 hours, more preferably, from 12 to 24 hours.

The CAR-transduced NK cells may be then cultured (optionally in the presence of IL-2 and/or IL-15 for simultaneous expansion) for a period sufficient for expression of the vector (i.e., formation of CAR-expressing NK cells), for example, from 1 to 14 days, more specifically from 1 to 3 days, from 3 to 5 days, from 5 to 7 days, from 7 to 10 days, or from 10 to 14 days.

Alternatively, the expression of the CAR on the NK cells may be carried out at the same time as the step of incubation of pyruvate and/or activating cytokines, i.e., the NK cells may be cultured in the presence of pyruvate and/or activating cytokines during the CAR transduction of NK cells and the culture for the vector expression.

Alternatively, the CAR may be transduced as described above into the provided NK cells, and then the CAR-expressing NK cells may be subjected to the step of incubation in the presence of pyruvate, and/or activating cytokines and/or target cells.

The construct of CARs may be designed appropriately, for example, depending on the disease of interest.

The CAR may comprise an extracellular target-binding domain (antigen recognition domain), a hinge region, a transmembrane domain that anchors the CAR to the cell membrane of the NK cell, and one or more intracellular domains that transmit activation signals.

The antigen recognition domain, especially the single-chain variable fragment (scFv), is well known in the art, and generated against an antigen of interest using a method already known in the art.

The step may further comprise a step of expanding the NK cells. The term "expanding (or expansion)" means increasing the number of NK cells (i.e., NK cells grow and proliferate).

The step of expanding the NK cells may be performed using a commercially available NK cell activation/expansion kit. For example, the step of expanding the NK cells may comprise incubating NK cells in a suitable cell culture medium that provides the necessary nutrients and growth factors for cell survival and proliferation (for example, MACS medium), preferably in the presence of cytokines such as IL-2 and/or IL-15 at an activating dose.

The step of expanding the NK cells may be performed prior to, at the same time as, or after the step of expressing a CAR on the surface of the provided NK cells, if the step is present. Alternatively or additionally, the step of expanding the NK cells may be performed prior to, at the same time as, or after the step of incubating the NK cells in the presence of pyruvate and optionally activating cytokines and/or targets.

The step of expanding the NK cells may be performed twice or more. As one example, the step of expansion may be performed prior to or at the same time as the step of expressing the CAR, if the step is present, in order to increase the efficiency of the CAR expression, and the step of expansion may be performed again prior to, at the same time as, or after the incubation in the presence of pyruvate and optionally activating cytokines and/or targets for the downstream application of the collected NK cells.

### Pharmaceutical composition

The invention also provides a pharmaceutical composition comprising NK cells obtained by the method as described above, and a pharmaceutically acceptable carrier.

The pharmaceutically acceptable carrier may comprise or may be a solvent, a diluent, an excipient, a dispersion medium, a coating, an antibacterial agent, an antifungal agent, isotonic, or absorption delaying agents. The pharmaceutically acceptable carrier may be appropriately selected depending on, for example, the desired format of the pharmaceutical composition (e.g., solid or liquid), which may be influenced by the administration mode of the pharmaceutical composition.

In the pharmaceutical composition, the NK cells may have an intracellular ATP (adenosine triphosphate) concentration of 1 to 10 fmol/cell. Assuming a cellular volume of 250 µm³, this range may correspond to an intracellular ATP concentration of 4 to 10 mM. The physiological intracellular ATP concentration is known to be approximately from 2 to 8 mM, as explained in Greiner JV, et al., Biology, 10(11):1166 (2021), for example.

The inventors have discovered that the extracellular pyruvate at supra-physiological concentration, after it is imported into the NK cells, contributes to ATP production by being oxidized into the TCA cycle. Thus, the intracellular ATP concentration may be an indicator characterizing NK cells potentiated by pyruvate. It should also be noted that some of the imported pyruvate is also converted to other substances, such as lactate.

The intracellular ATP concentration may be measured by, for example, luciferase assay, luminescence-based assay, bioluminescent-based assay, chemiluminescence-based assay, using any commercially available ATP assay kits.

In some embodiments, the intracellular ATP concentration of the NK cells in the pharmaceutical composition may be at least 1.5 times higher than that of NK cells which have not been subjected to the above method (in other words, non-potentiated NK cells). For example, the intracellular ATP concentration of the NK cells in the pharmaceutical composition may be 1.5 to 5 times, preferably 2 to 4 times higher than that of non-potentiated NK cells.

In some embodiments, the NK cells may express cytokines, preferably IFN-γ and MIP1-β.

IFN-γ is a cytokine that provides protection against diseases by acting directly on target cells or through activation of the host immune system. MIP1-β is a cytokine (specifically chemokine) that attracts a variety of immune cells to sites of microbial infection.

In some embodiments, at least 3% of the NK cells express IFN-γ in the pharmaceutical composition. For example, the proportion of the NK cells expressing IFN-γ may be from 3% to 6%, or more preferably from 4 to 5%.

In some embodiments, at least 40% of the NK cells express MIP1-β in the pharmaceutical composition. For example, the proportion of the NK cells expressing MIP1-β may be from 40% to 70%, or more preferably from 45 to 65%.

In some embodiments, at least 5% of the NK cells express surface cytotoxic markers, preferably CD107a.

CD107a is a surface cytotoxic marker which appears on the surface of the NK cells upon activation and that are involved in the degranulation capacities.

For example, the proportion of the NK cells expressing CD107a may be from 5 to 15%, more preferably from 8 to 15%.

The above characteristics of the intracellular ATP concentration and proportions of IFN-γ/MIP1-β/CD107a-expressing NK cells apply to the NK cells explained above in relation to the use of pyruvate for potentiating NK cells and the method for treating NK cells.

In some embodiments, the pharmaceutical composition may also comprise an additional component, for example, a ligand, agonist, or antagonist of an inhibitory receptor expressed by the target cell, an activator or inhibitor of a signaling pathway downstream of said inhibitory receptor, and/or a buffer such as neutral buffered saline, phosphate buffered saline and the like; a carbohydrate such as glucose, mannose, sucrose, dextrans or mannitol; a protein; a polypeptide or an amino acid such as glycine; antioxidants; a chelating agent such as EDTA or glutathione; an adjuvant (e.g. aluminium hydroxide); and a preservative.

The additional component may be appropriately selected depending on, for example, the desired format of the pharmaceutical composition (e.g., solid or liquid), which may be influenced by the administration mode of the pharmaceutical composition (which will be explained later). For example, when the pharmaceutical composition is in a liquid form, the pharmaceutical composition may comprise one or more of the following: sterile diluents such as water for injection, saline solution, preferably physiological saline, Ringer's solution, isotonic sodium chloride, fixed oils such as synthetic mono or diglycerides which may serve as a solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as EDTA; buffers such as acetates, citrates or phosphates and agents for the adjustment of tonicity such as sodium chloride or dextrose.

In some embodiments, the pharmaceutical composition may be an injectable pharmaceutical composition, which is preferably sterile.

The above pharmaceutical composition comprising NK cells and a pharmaceutically acceptable carrier may be administered for the treatment of cancer, an autoimmune disorder, an immune system disorder, and an infectious disease such as bacterial disease and viral disease.

The cancer, an autoimmune disorder, an immune system disorder, and an infectious disease such as bacterial disease and viral disease may be as described above in relation with the method for treating NK cells.

In some embodiments, the NK cells of the pharmaceutical composition for use in the treatment are derived from a subject, and the pharmaceutical composition may be intended for administration to the subject.

In other words, the pharmaceutical composition for use in treatment of the subject may be applied in autologous transplantation, using cells that are derived from the subject's own tissues. A benefit of an autologous strategy is that there is limited concern for immunogenic responses or transplant rejection.

In other embodiments, the NK cells of the pharmaceutical composition for use in the treatment may be derived from a subject, and the pharmaceutical composition may be intended for administration to a different subject.

In other words, the pharmaceutical composition for use in treatment of the different subject may be applied in allogenic transplantation. In this case, the subject and the different subject may be immunologically compatible, i.e., their MHC (major histocompatibility complex) proteins are very similar or identical (i.e., MHC-matched or partially mismatched). By "MHC" is meant the complex of genes which function in signaling between immune cells and antigens. An allogenic strategy can be advantageous especially when the patient does not have sufficient NK cells. In addition, such strategy can sometimes lead to a graft-vs-tumor effect (in which donor cells can be beneficial for the recipient by eliminating malignant cells) because the effect relies on the mismatch between the KIRs (Killer Immunoglobulin-like Receptors on NKs) and HLA (human leukocyte antigen).

The pharmaceutical composition may be intended for administration to the subject in a manner appropriate to the disease to be treated. The quantity and frequency of administration will be determined by factors such as the condition of the subject, and the type and severity of the subject's disease, and may be determined by the skilled practitioner, as is well known in the art, although appropriate dosages may also be determined by clinical trials.

As an example, the pharmaceutical composition may be administered to the subject at a concentration of from 1×10⁵ to 1×10⁸ cells/kg of body mass, for example, from 0.5×10⁶ to 1×10⁷ cells/kg of body mass.

Preferably, the mode of administration may be via injection or infusion, more preferably intravenous infusion. The intravenous infusion is known as a suitable mode of administration in cell therapy.

The pharmaceutical composition may be intended for administration to the subject in at least one route selected from intratumoral route, intramuscular route, intravenous route, and subcutaneous route, preferably intravenous route.

### Examples

The following examples illustrate the invention without limiting it.

### Example 1: Preparing NK cells

Peripheral blood samples from healthy subjects were obtained from the French blood bank (Etablissement Français du sang, Lyon, France).

Human PBMCs were separated from peripheral blood by Ficoll^{®} gradient centrifugation (Eurobio Laboratoires & AbCys) at room temperature. The PBMCs were kept at 4°C in complete medium overnight.

NK cells (CD56^{Dim} CD3) were isolated using an NK Cell Isolation Kit (Miltenyi Biotec) following the manufacturer's instructions, and then sorted (BD FACS ARIA II, purity > 99%, the following mAbs from eBioscience, BD Biosciences, Biolegend or Cell Signalling Technology were used: anti-CD56 (NCAM16.2), and anti-CD3 (UCHT1)). Freshly sorted NK cells were left to recover from the sorting overnight at 37°C with a non-activating dose of IL-15 (1 ng/ml, Peprotech) in RPMI 1640 medium additionally containing 10% FBS, 2mM HEPES, 2mM glutamine, 1mM pyruvate (sodium pyruvate) and 1% penicillin/streptomycin (HCL Technologies).

The following day, the cells were washed twice in PBS+0.5% BSA, and resuspended in RPMI 1640 medium powder (US biological) reconstituted following the manufacturer's instructions. The medium was supplemented with 1x Amino acid solution (R7331, Sigma), 2mM HEPES (Gibco) and 1% penicillin/ streptomycin (HCL Technologies). To avoid addition of undefined metabolites from FBS, the medium was complemented with 0.5% BSA. A non-activating dose of IL-15 (1 ng/ml) was present in all media.

### Example 2: Incubation of NK cells in the presence of pyruvate at different concentrations

The variation of the ATP concentration in NK cells incubated in the presence of varying concentrations of pyruvate (sodium pyruvate) was studied.

NK cells were prepared as in Example 1.

The isolated NK cells were seeded at 30,000 cells per well in 96-well plates, and incubated in the absence or in the presence of pyruvate at varying concentrations (0.01 to 10mM) for 4 hours, in the same medium as above.

The intracellular ATP concentration at each concentration of pyruvate was measured using a luciferase-based assay following the manufacturer's instructions (CellTiter-Glo^{®} 2.0 Cell Viability Assay and NAD/NADH-Glo Assay, Promega Corporation, Madison, WI). Luminescence was measured using a Tristar luminometer (Berthold).

The results are shown **Fig. 1****.**

**Fig. 1** shows that the intracellular ATP concentration increased dose-dependently with pyruvate concentration, and the intracellular ATP concentration was especially higher within the range of 0.35 to 10 mM of pyruvate, especially within the range of 1 to 10 mM of pyruvate.

The EC50 for the intracellular ATP production (the pyruvate concentration at which the ATP production is half-maximal) was 0.5 mM, which was well above the physiological blood concentration of pyruvate (i.e., 40 to 150 µM).

The inventors have also discovered that resting (non-stimulated) human NK cells are auxotroph for pyruvate, and that pyruvate production through glycolysis is limited due to the leak of intermediate products glycolysis into other metabolic pathways (data not shown).

This suggests that endogenous pyruvate may not be sufficient to sustain the TCA cycle for ATP production.

### Example 3: Incubation in the presence of pyruvate and target cells

### Measurement of cytotoxicity

The effect of varying concentrations of pyruvate on the cytotoxicity of the NK cells was studied.

NK cells were prepared as in Example 1, and were cultured in the absence or in the presence of pyruvate at varying concentrations (0.01 to 10 mM) for 3 hours and then co-cultured for 4 hours with K562 NanoLuc^{®}-expressing target cells. The supernatant was collected and bioluminescence was measured using a Tristar Instrument luminometer (Berthold) after addition of Furimazin (the substrate of NanoLuc). The percentage of cell lysis were calculated as explained in Hayek, S. et al., SLAS Discov. 24, 25-37 (2019).

The results are shown **Fig. 2****.**

In this Example, the activation of NK cells was further promoted by the co-culture with target cells (K562 cells).

The K562 lysis rate (%) showed an overall increase as the pyruvate concentration increased, and the K562 lysis rate within the range of 0.35 to 10 mM of pyruvate was higher than the K562 lysis rate within the physiological concentration of pyruvate in blood (40 to 150 µM). This indicates that the incubation of NK cells in the presence of pyruvate effectively improved the effector function of the NK cells.

In addition, the EC50 for the cytotoxicity of the NK cells (the pyruvate concentration at which the cytotoxicity is half-maximal) was 0.26 mM, almost twice higher than the physiological concentration.

Thus, the results of **Fig. 2** imply that the NK cells operate well below their maximal capacities at physiological pyruvate concentration.

### Measurement of marker expressions

The effect of varying concentrations of pyruvate on the expression of markers of NK cells was studied.

NK cells were prepared as in Example 1, and 1×10⁵ isolated NK cells were cultured in the absence or in the presence of pyruvate at varying concentrations (0.01 to 10mM) and a non-activating dose IL-15 (1 ng/ml) for 3 hours, then co-cultured for 4 hours with K562 cells at the Effector-Target ratio of 10:1 in the presence of Golgi Stop (BD Biosciences, protein transport inhibitor for intracellular accumulation pf the proteins) the last 3 hours.

The percentage of NK cells positive for CD107a, MIP1-β and IFN-γ was then determined by flow cytometry. Specifically, for CD107a, cell surface staining was performed during 30 min at 4°C in FACS buffer (PBS, 2% FBS, 2mM EDTA). Intracellular staining of MIP1-β and IFN-γ was performed with Cytofix/Cytoperm (BD Biosciences). The following mAbs from eBioscience, BD Biosciences, Biolegend or Cell Signalling Technology were used: anti-IFN-γ (4S.B3), anti-CD107a (H4A3), anti-MIP1-β (D21-1351). Sample acquisition was performed on an LSR Fortessa 5L (Becton-Dickinson). Data were analysed with FlowJo 10.5.0 software (Tree Star).

The results are shown in **Fig. 3**, specifically in **Fig. 3a** for CD107a⁺ NK cells, in **Fig. 3b** for IFN-γ⁺ NK cells, in **Fig. 3c** for MIP1-β⁺ NK cells.

For all the markers, the percentage of positive NK cells showed an overall increase as the pyruvate concentration increased.

In this Example, the activation of NK cells was further promoted by the co-culture with target cells (K562 cells).

According to the results shown in **Fig. 3**, the percentage of NK cells positive for each marker within the range of 0.35 to 10 mM of pyruvate concentration was higher than within the physiological concentration of pyruvate in blood (40 to 150 µM).

This indicates that the incubation of NK cells in the presence of pyruvate effectively potentiated the effector functions of NK cells.

The results of the above Examples overall demonstrate that, when isolated, NK cells possess spare capacities that can be triggered in the presence of pyruvate having a supra-physiological concentration. Thus, the incubation of NK cells with such pyruvate allows for efficient potentiation of the effector functions of the NK cells.

## Claims

1. Use of pyruvate for potentiating natural killer (NK) cells *in vitro.*

2. The use of claim 1, wherein the NK cells are potentiated by incubation in the presence of pyruvate at a concentration of from 0.35 to 10 mM, preferably 1 to 10 mM, more preferably from 1 to 5 mM.

3. The use of claim 1 or 2, wherein the NK cells are derived from a subject, preferably from peripheral blood mononuclear cells of the subject.

4. Method for treating *in vitro* NK cells, comprising the steps of:
- providing NK cells from a subject;
- incubating the NK cells in the presence of pyruvate at a concentration of from 0.35 to 10 mM, thereby potentiating the NK cells; and
- collecting the potentiated NK cells.

5. The method of claim 4, wherein incubating the NK cells in the presence of pyruvate is carried out for a period of from about 2 to 48 hours.

6. The method of claim 4 or 5, wherein the method further comprises incubating the NK cells in the presence of activating cytokines, the cytokines preferably comprising IL-12, IL-15, IL-18 and/or functional fragments thereof, preferably for a period of from about 2 to 48 hours, preferably from about 16 to 18 hours.

7. The method of any one of claims 4 to 6, wherein the step of providing NK cells from a subject comprises isolating the NK cells from peripheral blood mononuclear cells of the subject.

8. The method of any one of claims 4 to 7, wherein the subject suffers from a disease selected from cancer, an autoimmune disorder, an immune system disorder, and an infectious disease such as bacterial disease and viral disease.

9. The method of any one of claims 4 to 8, wherein the method further comprises a step of expressing a chimeric antigen receptor on the surface of the NK cells prior to, at the same time as, or after the step of incubation.

10. A pharmaceutical composition comprising NK cells obtained by the method of any one of claims 4 to 9, and a pharmaceutically acceptable carrier.

11. The pharmaceutical composition of claim 10, wherein the NK cells have an intracellular ATP concentration of 1 to 10 fmol/cell.

12. The pharmaceutical composition of claim 10 or 11, wherein the NK cells express cytokines, preferably IFN-γ and MIP1-β, and/or surface cytotoxic markers, preferably CD107a, more preferably at least 3% of the NK cells expressing the IFN-γ, and/or at least 40% of the NK cells expressing MIP1-β, and/or at least 5% of the NK cells expressing CD107a.

13. A pharmaceutical composition comprising NK cells obtained by the method of any one of claims 4 to 9 and a pharmaceutically acceptable carrier, for use in the treatment of cancer, an autoimmune disorder, an immune system disorder, and an infectious disease such as bacterial disease and viral disease.

14. The pharmaceutical composition for use according to claim 13, wherein the NK cells have an intracellular ATP concentration of 1 to 10 fmol/cell or higher.

15. The pharmaceutical composition for use according to claim 13 or 14, wherein the pharmaceutical composition is intended for administration to the subject, preferably via intravenous infusion, and wherein preferably the NK cells are derived from the same subject.
